Europäisches Patentamt

European Patent Office

Office européen des brevets

(19)

(11) **EP 1 142 901 B1**

(12) **FASCICULE DE BREVET EUROPEEN**

(45) Date de publication et mention
de la délivrance du brevet:
**07.05.2003 Bulletin 2003/19**

(51) Int Cl.⁷: **C07H 15/04**, B01F 17/56,
C11D 3/22

(21) Numéro de dépôt: **01400748.8**

(22) Date de dépôt: **22.03.2001**

(54) **Dérivés de polyxylosides, procédé pour leur préparation, composition en comportant et utilisation comme agents tensioactifs**

Polyxylosidderivate, Verfahren zu deren Herstellung, Zusammensetzung und Verwendung als Tensid

Polyxyloside derivatives, method of preparation, composition and use as surfactant

(84) Etats contractants désignés:
**DE ES FR GB IT NL SE**

(30) Priorité: **06.04.2000 FR 0004414**

(43) Date de publication de la demande:
**10.10.2001 Bulletin 2001/41**

(73) Titulaire: **SOCIETE D'EXPLOITATION DE PRODUITS POUR LES INDUSTRIES CHIMIQUES, S.E.P.P.I.C.**
**75321 Paris Cédex 07 (FR)**

(72) Inventeurs:
• **Milius, Alain**
  **06000 Nice (FR)**
• **Boiteux, Jean-Pierre**
  **81710 Saix (FR)**
• **Rolland, Hervé**
  **81100 Castres (FR)**
• **Tabacchi, Guy**
  **81100 Castres (FR)**

(74) Mandataire: **Conan, Philippe Claude et al**
**L'Air Liquide**
**D.S.P.I.**
**75, quai d'Orsay**
**75321 Paris Cedex 07 (FR)**

(56) Documents cités:
  EP-A- 0 092 355          EP-A- 0 387 912
  DE-A- 4 311 159          US-A- 5 681 949

• **C. K. DE BRUYNE, F. G. LOONTIENS: "Synthesis of alkyl-beta-D-xylopyranosides in the presence of mercuric salts" NATURE, vol. 209, 1966, pages 396-397, XP002154836**
• **A. KUSANO ET AL.: "Studies on the constituents of Cimicifuga species. XVIII. Four new xylosides from the aerial parts of Cimicifuga simplex WORMSK" CHEM. PHARM. BULL., vol. 44, no. 1, 1996, pages 167-172, XP002154837**

**Description**

La présente invention concerne de nouveaux composés dérivés de sucre, les procédés pour leur préparation et leurs utilisations comme agents tensioactifs.

Le brevet américain publié sous le numéro 5,681,949, enseigne que les composés alkyl polyglycosides ayant une chaîne alkyle ramifiée, l'une des ramifications comportant entre deux et cinq atomes de carbone, l'autre ramification comportant de quatre à sept atomes de carbone et la somme des atomes de carbone de ces deux ramifications étant comprise entre sept et onze, présentent des propriétés mouillantes et nettoyantes améliorées par rapport aux alkyl polyglycosides connus à l'époque.

Lors de ses recherches, ayant pour objectif de préparer des agents tensioactifs capables de conduire à des émulsions vésiculaires ou à des micelles inverses, la demanderesse s'est aperçue que certains de ces composés bicaténaires permettaient d'atteindre ce type d'émulsions.

C'est pourquoi l'invention a pour objet un composé de formule (I) :

$$RO\text{-}(X)_p \qquad\qquad (1)$$

dans laquelle :

p représente un nombre décimal compris entre 1 et 5 et
X représente le reste du xylose, et
R représente un radical alkyl ramifié :

$$CH(C_nH_{2n+1})(C_mH_{2m+1})\text{-}CH_2\text{-}$$

dans lequel m est un nombre entier compris entre 6 et 18, n est un nombre entier compris entre 4 et 18 et la somme n + m est supérieure ou égale à 12.

La structure oligomérique $(X)_p$, peut se présenter sous toute forme d'isomérie, qu'il s'agisse d'isomérie optique, d'isomérie géométrique ou d'isomérie de position; elle peut aussi représenter un mélange d'isomères.

Dans la formule (I), le groupe R-O- est lié à X par le carbone anomérique du reste saccharide, de manière à former une fonction acétal.

p, qui représente le degré moyen de polymérisation du saccharide, est plus particulièrement compris entre 1 et 2,5 et tout particulièrement entre 1 et 2,0.

Selon un premier aspect particulier de la présente invention, celle-ci a pour objet un composé de formule (I), telle que définie précédemment, dans laquelle n et m sont des nombres pairs.

Selon un deuxième aspect particulier de la présente invention, celle-ci a pour objet un composé de formule (I), telle que définie précédemment, dans laquelle la somme n + m est égale à 12. Dans ce cas, R représente plus particulièrement un des radicaux 2-butyl décyle (m=4, n=8) ou 2-hexyl octyle (m=6, n=6).

Selon un troisième aspect particulier de la présente invention, celle-ci a pour objet un composé de formule (I), telle que définie précédemment, dans laquelle la somme n + m est supérieure ou égale à 14. Dans ce cas, R représente plus particulièrement un des radicaux 2-hexyl décyle (n=6, m=8), 2-hexyl dodécyle (m=6, n=10), 2-octyle décyle (m=8, n=8), 2-octyl dodécyle (m=8, n=10), 2-décyl tétradécyle (m=10, n=12), 2-dodécyl hexadécyle (m=12, n=14), 2-tétradécyl octadécyle (m=14, n=16), 2-tétradécyl eicosyle (m=14, n=18), 2-hexadécyl octadécyle (m=16, n=16) ou 2-hexadécyl eicosyle (m=16, n=18).

L'invention a aussi pour objet, un procédé de préparation d'un composé de formule (I), telle que définie précédemment, caractérisé en ce que le xylose de formule (II) :

$$HO\text{-}X \qquad\qquad (II)$$

est mis à réagir avec un excès d'alcool de formule (III) :

$$ROH, \qquad\qquad (III)$$

pour former, après élimination de l'alcool gras de formule (III) n'ayant pas réagi, le composé de formule (I).

Dans le procédé tel que défini ci-dessus, la réaction de formation du composé de formule (III) est effectuée en présence de catalyseurs acides forts, tels que par exemple les acides minéraux comme l'acide sulfurique, l'acide hypophosphoreux ou un mélange de ces acides.

Selon une variante du procédé tel que défini précédemment, le xylose de formule (II) est mis à réagir avec un alcool de formule (IV):

$$R_1\text{-OH}$$

dans laquelle $R_1$ comporte de 1 à 4 atomes de carbone et plus particulièrement, avec le butanol, pour conduire à l'acétal de formule (V) :

$$R_1O\text{-}(X)_p, \hspace{4cm} \text{(V)}$$

acétal de formule (V), qui subit ensuite une trans-acétalisation par un excès d'alcool de formule (III) avec distillation de l'alcool de formule (IV) formé puis élimination de l'alcool de formule (III) n'ayant pas réagi.

Dans le procédé et sa variante, tels que décrits précédemment, l'élimination de l'alcool de formule (III) n'ayant pas réagi, est effectuée selon des méthodes connues de l'homme du métier comme, par exemple, la distillation, la distillation sur film mince, la distillation moléculaire ou l'extraction par solvants.

Selon un autre aspect de la présente invention, celle-ci a pour objet une composition (A), consistant en un mélange d'au moins deux composés de formule (I), susceptible d'être obtenue par le procédé ou sa variante, tel qu'ils sont définis ci-dessus, en mettant en oeuvre un mélange d'alcools de formules (III), à la place d'un seul de ces alcools de formule (III).

Selon un autre aspect de la présente invention celle-ci a pour objet une composition (B) caractérisée en ce qu'elle comprend

plus de 0% en poids et moins de 100% en poids, de préférence de 1% à 60% en poids, d'un composé de formule (I) ou d'un mélange de composés de formule (I), telle que définie précédemment et

plus de 0% en poids et moins de 100% en poids, de préférence de 40% à 99% en poids, d'un composé de formule (III) ou d'un mélange de composés de formule (III), telle que définie précédemment.

La composition B, telle que définie ci-dessus, est préparée par exemple, en faisant réagir un excès de composés de formules (III) avec le xylose selon le procédé ou sa variante, tels qu'ils sont définis ci-dessus, sans élimination des alcools de formules (III) n'ayant pas réagi. Elle peut aussi être obtenue en mélangeant un alcool de formule (III) ou un mélange d'alcools de formules (III), avec un composé de formule (I) ou un mélange de composés de formule (I).

La chaîne R ou les chaînes R du composé de formule (III) ou des composés de formules (III) sont de préférence identiques à celles du composé de formule (I) ou des composés de formules (I).

Selon un dernier aspect de la présente invention, celle-ci a pour objet l'utilisation d'un composé de formule (I), d'une composition (A) ou d'une composition (B), telle que décrits précédemment, comme agent de surface ou agent tensioactif et, plus particulièrement, l'utilisation comme agent émulsionnant lorsque la somme n + m est supérieure ou égale 14 et comme agent moussant, agent mouillant, agent détergent ou agent solubilisant des agents anti-mousse, lorsque la somme n+ m est inférieure à 14.

Les exemples suivants illustrent l'invention, sans toutefois la limiter.

### Exemple 1 Acétalisation du xylose avec de l'ISOFOL™18 en excès (Composition $B_1$)

On chauffe à 90°C, dans un réacteur, un mélange d'alcools comprenant majoritairement du 2-hexyl dodécanol et du 2-octyl dodécanol, commercialisé sous le nom ISOFOL™18, puis on ajoute sous agitation du xylose dans le rapport stoechiométrique Xylose / alcools = 1/6 et laisse réagir pendant 4 heures en présence d'un catalyseur acide.

Après refroidissement, neutralisation et filtration, on obtient un mélange $B_1$ de xylosides et d'alcools gras, peu coloré et répondant aux caractéristiques suivantes :

| | |
|---|---|
| Indice d'acide ($I_A$) | 0,05 |
| Indice d'hydroxyte ($I_{OH}$) | 241,5 |
| Taux d'alcools libres dans le mélange final $B_1$ : | 84,9 % |

(suite)

| Taux d'alkyl polyxylosides dans le mélange final $B_1$ : | 15,1% |
|---|---|

### Exemple 2 : Acétalisation du xylose avec de l'ISOFOL™36 (Compostion $B_2$)

On mélange sous agitation du butanol en excès et du xylose en présence d'un catalyseur acide à 100°C sous pression réduite de façon à évaporer l'eau formée. Au bout de deux heures on ajoute de l'ISOFOL™36, qui est constitué majoritairement de 2-hexadécyl eicosanol (m=16, n=18) en excès et l'on laisse réagir pendant 7 heures en distillant le butanol libéré.

Après refroidissement, neutralisation et filtration, on obtient un tensioactif émulsionnant correspondant à une composition B telle que définie précédemment, comprenant environ 60% de 2-hexadécyl eicosanol libre et environ 40% d'un produit de formule(I) dans laquelle R représente un radical 2-hexadécyl eicosyle (composition $B_2$).

### Préparation d'une composition de l'état de la technique (composition $C_1$)

On chauffe à 90°C, dans un réacteur, de l'ISOFOL™18, puis on ajoute sous agitation du glucose dans le rapport stoechiométrique glucose / alcools = 1/6 et laisse réagir pendant 4 heures, en présence d'un catalyseur acide.

Après refroidissement, neutralisation et filtration, on obtient un mélange $C_1$ d'alkylglucosides et d'alcools gras.

### Etude de stabilité des émulsions

On prépare par simple mélange à chaud des différents constituants, les émulsions $E_1$, $E_2$ et $E_3$, avec, comme agent émulsionnant la composition $B_1$ de l'exemple 1, et les émulsions $F_1$, $G_1$, $F_2$, $G_2$, $F_3$ et $G_3$, avec comme agent émulsionnant la composition $C_1$.

On observe, que les émulsions $E_1$ à $E_3$ préparées avec la composition selon l'invention, sont plus stables que les émulsions préparées avec la même concentration de composition $C_1$ de l'état de technique.

Les résultats sont consignés dans le tableau suivant (le résultat ++ indique que l'on n'a observé aucun déphasage après avoir maintenu l'émulsion pendant un mois à 40°C. Le résultat - indique que l'on a observé un déphasage de l'émulsion après 15 jours à 40°C)

| Constituants (% pondéraux) | Emulsions | | | | | | | | |
|---|---|---|---|---|---|---|---|---|---|
| | $E_1$ | $F_1$ | $G_1$ | $E_2$ | $F_2$ | $G_2$ | $E_3$ | $F_3$ | $G_3$ |
| $B_1$ | 3 | 0 | 0 | 3 | 0 | 0 | 3 | 0 | 0 |
| $C_1$ | 0 | 3 | 5 | 0 | 3 | 5 | 0 | 3 | 5 |
| PRIMOL™352 | 5 | 5 | 5 | 0 | 0 | 0 | 0 | 0 | 0 |
| TRIGLY™5545 | 0 | 0 | 0 | 5 | 5 | 5 | 0 | 0 | 0 |
| LANOL™99 | 0 | 0 | 0 | 0 | 0 | 0 | 5 | 5 | 5 |
| SIMULGEL™EG | 1 | 1 | 1 | 1 | 1 | 1 | 1 | 1 | 1 |
| Eau | qs | qs | qs | qs | qs | qs | qs | qs | qs |
| Conservateur | qs | qs | qs | qs | qs | qs | qs | qs | qs |
| Stabilité à 40°C | ++ | - | ++ | ++ | - | ++ | ++ | - | ++ |

Ce résultat indique que l'on peut préparer des émulsions stables, en mettant en oeuvre moins d'alkylpolyxylosides que d'alkylpolyglucosides émulsionnants.

**Revendications**

1.   Composé de formule (I) :

$$RO\text{-}(X)_p \qquad\qquad (I)$$

dans laquelle :

p représente un nombre décimal compris entre 1 et 5 et
X représente le reste du xylose, et
R représente un radical alkyl ramifié :

$$CH(C_nH_{2n+1})(C_mH_{2m+1})\text{-}CH_2\text{-}$$

dans lequel m est un nombre entier compris entre 6 et 18, n est un nombre entier compris entre 4 et 18 et la somme n + m est supérieure ou égale à 12.

2.   Composé de formule (I), telle que définie à la revendication 1, dans laquelle p, qui représente le degré moyen de polymérisation du saccharide, est compris entre 1 et 2,5 et plus particulièrement entre 1 et 2,0.

3.   Composé de formule (I), telle que définie à l'une des revendications 1 ou 2, dans laquelle n et m sont des nombres pairs.

4.   Composé de formule (I), telle que définie à l'une quelconques des revendications 1 à 3, dans laquelle la somme n + m est égale à 12.

5.   Composé de formule (I), telle que définie à la revendication 4, dans laquelle R représente un des radicaux 2-butyl décyle ou 2-hexyl octyle.

6.   Composé de formule (I), telle que définie à l'une quelconque des revendications 1 ou 2, dans laquelle la somme n + m est supérieure ou égale à 14.

7.   Composé de formule (I), telle que définie à la revendication 6, dans laquelle R représente un des radicaux 2-hexyl décyle, 2-hexyl dodécyle, 2-octyl décyle, 2-octyl dodécyle, 2-décyl tétradécyle, 2-dodécyl hexadécyle, 2-tétradécyl octadécyle, 2-tétradécyl eicosyle, 2-hexadécyl octadécyle ou 2-hexadécyl eicosyle.

8.   Procédé de préparation d'un composé de formule (I), telle que définie à l'une quelconque des revendications 1 à 7, **caractérisé en ce que** le xylose de formule (II):

$$HO\text{-}X \qquad\qquad (II)$$

est mis à réagir avec un excès d'alcool de formule (III) :

$$ROH\ (III), \qquad\qquad (III)$$

pour former, après élimination de l'alcool gras de formule (III) n'ayant pas réagi, le composé de formule (I).

9.   Variante du procédé tel que défini à la revendication 8, selon laquelle le xylose de formule (II) est mis à réagir avec un alcool de formule (IV):

$$R_1\text{-OH}$$

dans laquelle $R_1$ comporte de 1 à 4 atomes de carbone et plus particulièrement, avec le butanol, pour conduire à l'acétal de formule (V) :

$$R_1O\text{-}(X)_p, \hspace{4cm} (V)$$

acétal de formule (V), qui subit ensuite une trans-acétalisation par un excès d'alcool de formule (III) avec distillation de l'alcool de formule (IV) formé puis élimination de l'alcool de formule (III) n'ayant pas réagi.

10. Composition (A), consistant en un mélange d'au moins deux composés de formule (I), telle que définie à l'une quelconque des revendications 1 à 7, susceptible d'être obtenue en mettant à réagir le xylose avec un mélange d'alcools de formules (III).

11. Composition (B) **caractérisée en ce qu'**elle comprend :

plus de 0% en poids et moins de 100% en poids, de préférence de 1% à 60% en poids, d'un composé de formule (I) ou d'un mélange de composés de formule (I), telle que définie à l'une quelconque des revendications 1 à 7 et,
plus de 0% en poids et moins de 100% en poids, de préférence de 40% à 99% en poids, d'un composé de formule (III) ou d'un mélange de composés de formule (III).

12. Utilisation d'un composé de formule (I), d'une composition (A) ou d'une composition (B), telle que définie à l'une quelconque des revendications 1 à 7 et 10 ou 11, comme agent de surface ou agent tensioactif.

13. Utilisation d'un composé de formule (I), d'une composition (A) ou d'une composition (B), pour lesquels la somme $n + m$ est supérieure ou égale 14, comme agent émulsionnant.

14. Utilisation d'un composé de formule (I), d'une composition (A) ou d'une composition (B), pour lesquels la somme $n + m$ est inférieure à 14, comme agent moussant, agent mouillant, agent détergent ou agent solubilisant des agents anti-mousse.

**Patentansprüche**

1. Verbindung der Formel (I):

$$RO\text{-}(X)_p \hspace{4cm} (I)$$

worin:

$p$ für eine Dezimalzahl zwischen 1 und 5 steht,
X für einen Xyloserest steht und
R für einen verzweigten Alkylrest

$$CH(C_nH_{2n+1})(C_mH_{2m+1})\text{-}CH_2\text{-}$$

worin m eine ganze Zahl zwischen 6 und 18 und n eine ganze Zahl zwischen 4 und 18 bedeutet und die Summe $n + m$ größer gleich 12 ist, steht.

2. Verbindung der Formel (I) nach Anspruch 1, in der p, der mittlere Polymerisationsgrad des Saccharids, zwischen 1 und 2,5 und insbesondere zwischen 1 und 2,0 liegt.

**3.** Verbindung der Formel (I) nach Anspruch 1 oder 2, in der n und m für gerade Zahlen stehen.

**4.** Verbindung der Formel (I) nach einem der Ansprüche 1 bis 3, in der die Summe n + m gleich 12 ist.

**5.** Verbindung der Formel (I) nach Anspruch 4, in der R für einen 2-Butyldecyl- oder 2-Hexyloctylrest steht.

**6.** Verbindung der Formel (I) nach Anspruch 1 oder 2, in der die Summe n + m größer gleich 14 ist.

**7.** Verbindung der Formel (I) nach Anspruch 6, in der R für einen 2-Hexyldecyl-, 2-Hexyldodecyl-, 2-Octyldecyl-, 2-Octyldodecyl-, 2-Decyltetradecyl-, 2-Dodecylhexadecyl-, 2-Tetradecyloctadecyl-, 2-Tetradecyleicosyl-, 2-Hexadecyloctadecyl- oder 2-Hexadecyleicosylrest steht.

**8.** Verfahren zur Herstellung einer Verbindung der Formel (I) nach einem der Ansprüche 1 bis 7, **dadurch gekennzeichnet, daß** man die Xylose der Formel (II):

$$HO\text{-}X \qquad\qquad (II)$$

mit einem Überschuß an Alkohol der Formel (III):

$$ROH \qquad\qquad (III)$$

umsetzt, wobei man nach Entfernung des nicht umgesetzten Fettalkohols der Formel (III) die Verbindung der Formel (I) erhält.

**9.** Variante des Verfahrens nach Anspruch 8, bei dem man die Xylose der Formel (II) mit einem Alkohol der Formel (IV):

$$R_1OH$$

worin $R_1$ 1 bis 4 Kohlenstoffatome enthält, und insbesondere mit Butanol zu einem Acetal der Formel (V):

$$R_1O\text{-}(X)_p \qquad\qquad (V)$$

umsetzt, dieses dann einer Umacetalisierung mit einem Überschuß an Alkohol der Formel (III) unter Abdestillieren des gebildeten Alkohols der Formel (IV) unterwirft und danach den nicht umgesetzten Alkohol der Formel (III) entfernt.

**10.** Zusammensetzung (A), bestehend aus einem Gemisch aus mindestens zwei Verbindungen der Formel (I) nach einem der Ansprüche 1 bis 7, die durch Umsetzung von Xylose mit einem Gemisch von Alkoholen der Formel (III) erhältlich ist.

**11.** Zusammensetzung (B), **dadurch gekennzeichnet, daß** sie:

mehr als 0 Gew.-% und weniger als 100 Gew.-%, vorzugsweise 1 bis 60 Gew.-%, einer Verbindung der Formel (I) oder eines Gemischs von Verbindungen der Formel (I) nach einem der Ansprüche 1 bis 7 und

mehr als 0 Gew.-% und weniger als 100 Gew.-%, vorzugsweise 40 bis 99 Gew.-%, einer Verbindung der Formel (III) oder eines Gemischs von Verbindungen der Formel (III)

enthält.

**12.** Verwendung einer Verbindung der Formel (I), einer Zusammensetzung (A) oder einer Zusammensetzung (B) nach einem der Ansprüche 1 bis 7 und 10 oder 11 als Tensid.

13. Verwendung einer Verbindung der Formel (I), einer Zusammensetzung (A) oder einer Zusammensetzung (B), für die die Summe n + m größer gleich 14 ist, als Emulgiermittel.

14. Verwendung einer Verbindung der Formel (I), einer Zusammensetzung (A) oder einer Zusammensetzung (B), für die die Summe n + m kleiner 14 ist, als Schaummittel, Netzmittel, Detergens oder Mittel zum Lösen von Entschäumern.

**Claims**

1. Compound of formula (I):

$$RO\text{-}(X)_p \qquad (I)$$

in which:

p represents a decimal number between 1 and 5 and
X represents a xylose residue, and
R represents a branched alkyl radical:

$$CH(C_nH_{2n+1})(C_mH_{2m+1})\text{-}CH_2\text{-}$$

in which m is an integer between 6 and 18, n is an integer between 4 and 18 and the sum n + m is greater than or equal to 12.

2. Compound of formula (I) as defined in Claim 1, in which p, which represents the average degree of polymerization of the saccharide, is between 1 and 2.5 and more particularly between 1 and 2.0.

3. Compound of formula (I) as defined in either of Claims 1 and 2, in which n and m are even numbers.

4. Compound of formula (I) as defined in any one of Claims 1 to 3, in which the sum n + m is equal to 12.

5. Compound of formula (I) as defined in Claim 4, in which R represents a 2-butyldecyl radical or a 2-hexyloctyl radical.

6. Compound of formula (I) as defined in either one of Claims 1 and 2, in which the sum n + m is greater than or equal to 14.

7. Compound of formula (I) as defined in Claim 6, in which R represents a 2-hexyldecyl radical, a 2-hexyldodecyl radical, a 2-octyldecyl radical, a 2-octyldodecyl radical, a 2-decyltetradecyl radical, a 2-dodecylhexadecyl radical, a 2-tetradecyloctadecyl radical, a 2-tetradecyleicosyl radical, a 2-hexadecyloctadecyl radical or a 2-hexadecyleicosyl radical.

8. Process for preparing a compound of formula (I) as defined in any one of Claims 1 to 7, **characterized in that** the xylose of formula (II):

$$HO\text{-}X \qquad (II)$$

is reacted with an excess of alcohol of formula (III):

$$ROH \qquad (III)$$

to form, after removal of the unreacted fatty alcohol of formula (III), the compound of formula (I).

9. Variant of the process as defined in Claim 8, in which the xylose of formula (II) is reacted with an alcohol of formula (IV):

$$R_1\text{-OH}$$

in which $R_1$ contains from 1 to 4 carbon atoms, and more particularly with butanol, to give the acetal of formula (V):

$$R_1O\text{-}(X)_p, \hspace{4cm} (V)$$

this acetal of formula (V) then undergoing a transacetalization with an excess of alcohol of formula (III) with distillation of the alcohol of formula (IV) formed, followed by removal of the unreacted alcohol of formula (III).

10. Composition (A), consisting of a mixture of at least two compounds of formula (I) as defined in any one of Claims 1 to 7, which may be obtained by reacting xylose with a mixture of alcohols of formula (III).

11. Composition (B), **characterized in that** it comprises:

   more than 0% by weight and less than 100% by weight, preferably from 1% to 60% by weight, of a compound of formula (I) or of a mixture of compounds of formula (I), as defined in any one of Claims 1 to 7, and
   more than 0% by weight and less than 100% by weight, preferably from 40% to 99% by weight, of a compound of formula (III) or of a mixture of compounds of formula (III).

12. Use of a compound of formula (I), of a composition (A) or of a composition (B), as defined in any one of Claims 1 to 7, 10 and 11, as a surface agent or surfactant.

13. Use of a compound of formula (I), of a composition (A) or of a composition (B), for which the sum $n + m$ is greater than or equal to 14, as an emulsifier.

14. Use of a compound of formula (I), of a composition (A) or of a composition (B), for which the sum $n + m$ is less than 14, as a foaming agent, wetting agent, detergent or antifoam solubilizing agent.